# EUROPEAN PATENT APPLICATION

(11) **EP 1 053 731 A2**
(43) Date of publication of application: **22.11.2000**
(21) Application number: 00830261.4
(22) Date of filing: 06.04.2000
(51) Int. Cl.: A61F 13/15

(54) **Sanitary pad**

(30) Priority: 06.04.1999 IT RM990067 U
(71) Applicant: Carboni, Giovanna, 07040 S. Givanni SS (IT)
(72) Inventor: Carboni, Giovanna, 07040 S. Givanni SS (IT)
(74) Representative: Leone, Mario

(57) **Abstract**

Anti-vaginitis sanitary pad (1; 100) comprising a substantially flat elongated body (1; 100), having a longitudinal dimension (2) and a transverse dimension (3) greater than the thickness, which elongated body having a fluid-absorbing multi-layer structure providing a fluid-permeable top layer (11), apt to contact the user's skin, an intermediate absorbent core (12) and a fluid-impermeable bottom layer (13, 103), apt to be adhered to a panty, wherein the elongated body comprises a semirigid material element (8; 80; 800), arranged in a substantially centred position with respect to the longitudinal and transverse dimensions.

## Description

The present invention relates to a sanitary pad. In particular, it relates to a pad comprising a substantially flat elongated body, having a longitudinal dimension and a transverse dimension larger than the thickness, which elongated body has a multi-layer structure for absorbing organic fluids, said structure providing a fluid permeable top layer, apt to contact the user's skin, an intermediate absorbent core and a bottom layer, fluid impermeable and apt to be adhered to a panty.

Trousers, tights, underwear and the like are usually provided with seams at the crotch area. These seams tend to insert within the female external genital apparatus, often exerting markedly localised pressures and causing rubbing. Not only such actions of the seams onto the external genital apparatus are very bothersome, but they can also worsen, or even cause, inflammations or irritations of the vaginal canal and/or of the external genitourinary system in general, like, e.g., vaginitids.

Of course, the above-mentioned actions are more accentuated in the user wearing close-fitting garments. Anyway, they can occur regardless of the specific garment in wear, whenever motions causing a tighter adherence of the garnment to the user's body are performed. For instance, these actions can take place while seating, making sports, cycling or riding a motorbyke.

In order to overcome the above-mentioned problems, many women are wary of using close-fitting garments, at least while the inflamed or irritated state persists. It will be understood that such a wariness could be stressful and hinder the free course of the user's daily life. Above all, moreover, it is not resolutive.

The technical problem underlying the present invention is that of providing a sanitary pad allowing to overcome the above-mentioned drawbacks.

This problem is solved by a sanitary pad, comprising a substantially flat elongated body, having a longitudinal dimension and a transverse dimension larger than the thickness, which elongated body has a multi-layer structure for absorbing organic fluids, said structure providing a fluid permeable top layer, apt to contact the user's skin, an intermediate absorbent core and a bottom layer, fluid impermeable, apt to be adhered to the panty, characterised in that said elongated body, comprises an element of a semirigid material, arranged in a substantially centred position with respect to said longitudinal and transverse dimensions.

The present invention provides some relevant advantages. The main advantage lies in the fact that said element of a semirigid material prevents the insertion between the female external genital apparatus of the seams formed at the crotch, causing the above-mentioned injurious actions.

Other advantages, features and operation modes of the present invention will be made evident in the following detailed description of some embodiments thereof, given by way of example and not for limitative purposes. Reference will be made to the figures of the annexed drawings, wherein:
FIG. 1 is a partially sectional perspective view of a first embodiment of the sanitary pad according to the present invention;
FIG. 2 is an exploded perspective view of the sanitary pad of FIG. 1;
FIG. 3 is a partially sectional perspective view of an element of a semirigid material of a second embodiment of the sanitary pad according to the present invention; and
FIG. 4 is a partially exploded perspective view of a third embodiment of the sanitary pad according to the present invention.

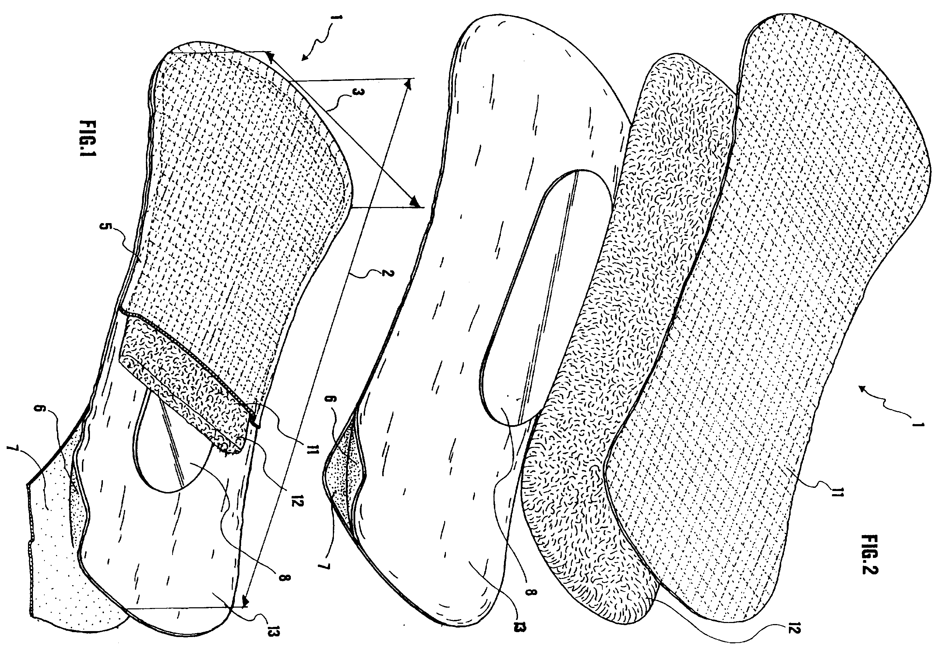

Referring to FIG. 1, a disposable sanitary pad, globally indicated with 1, comprises a substantially flat elongated body, that is also indicated with 1. Thus, such elongated body 1 has two dimensions, larger than the thickness thereof, i.e. a greater longitudinal dimension, indicated by arrow 2 in FIG. 1, and a smaller transverse dimension, indicated by arrow 3.

The elongated body 1 provides a multi-layer structure for absorbing organic fluids like vaginal discharge, urine, and the like. Such multi-layer structure will hereinafter be better understood by referring also to FIG. 2.

According to said multi-layer structure, the elongated body 1 comprises a top layer 11, permeable to the above-mentioned organic discharge and apt to contact the user's skin. As it is well-known to those skilled in the art, such top layer 11 will preferably be made of a soft, flexible material, not irritating the user's skin. For instance, such top layer could be made of polyester, polypropylene, nylon and/or rayon. The top layer 11 can have perforations, visible even to a naked eye, sketched in FIGS. 1 and 2.

Always referring to the above figures, from top to bottom, the multi-layer structure of the sanitary pad 1 further provides an inner absorbent core 12. Such absorbent core 12 is typically made of a highly compressible material, soft and not irritating the user's skin. In this instance as well, a person skilled in the art will be aware of several suitable materials therefor, such as, e.g., cotton wool, fluid-absorbing sponges, polymeric fibres, polyethylene terephtalate, fluid-absorbing polymeric gels or any other equivalent material or material combination.

The multi-layer structure also provides a bottom layer 13, apt to be adhered to a panty. Of course, in order to avoid garment staining such bottom layer 13 must be impermeable to organic fluids. Analogously to the above-mentioned, a person skilled in the art will be aware of several suitable materials for the bottom layer 13, like, e.g., polyethylene film or laminated tissues.

An adhesive 6, is provided onto the lower face of the bottom layer 13, only partially visible in the Figures, in order to bond the elongated body 1 onto the underwear. In the present embodiment, in order to ensure maximum adherence such adhesive 6 is applied over the entire area of said lower face.

The sanitary pad 1 is provided to the end user with a protective release band 7 adhered onto the lower face of the bottom layer 13. Such protective release layer 7 is precisely apt to protect the adhesive 6 from dust and dirt in general and to avoid its drying, or an attachment to unintended surfaces prior to fastening onto the panty. As it is well-known, such protective release layer 7 is peeled off the bottom layer 13 of the elongated body 1 and disposed off when attaching the sanitary pad 1 onto the panty.

The longitudinal dimension 2 of the elongated body 1 is such as to allow the pad to follow the contour of the user's pelvis, in order to completely shield the panty from the external genital apparatus, and therefore from the fluid discharge thereof.

The three layers of the sanitary pad 1 of the present embodiment are joined therebetween by a glueing 5, carried out at the perimeter of the elongated body 1. Such bonding 5 can be carried out, e.g., with an adhesive, by crinkling or by heat seal.

Of course, alternative embodiments could provide a different type of connection among the aforesaid layers. For instance, the latter could be joined thereamong by means of glue depositions, e.g., an elastomer adhesive, applied over the entire area of layer overlapping, instead of only at a perimeter area thereof, as in the present embodiment.

All the hereto mentioned features of the sanitary pad 1 will be well-known to those skilled in the art, hence a further description thereof will be omitted.

According to the invention, the elongated body 1 further comprises a substantially flat element of a semirigid material 8, arranged in a substantially centred position with respect to the longitudinal and transverse dimensions 2 and 3 of the elongated body 1.

Here, the term 〈〈semirigid〉〉 is intended to denote an element that is less deformable than the other layers of the pad 1 already disclosed, and therefore such as to prevent the insertion between the female external genital apparatus of the seams of trousers, tights and/or underwear. Suitable materials for making this element can be plastic materials selected from a group comprising Plexiglas and polyethylene, or light metal materials such as aluminium. Of course, the semirigid element 8 could also be made of a combination of the above-mentioned materials.

When pad 1 in worn, the element of a semirigid material 8 lies at the vaginal opening, due to the above-mentioned centred arrangement thereof with respect to the elongated body 1. Therefore, this element functions as a shield against the insertion between the female external genital apparatus of the seams of trousers, underwear and the like, preventing the pad 1 to deform for allowing such insertion.

Usually, these materials are organic fluid-impermeable.

In the present embodiment, the semirigid element 8 is arranged between the absorbent core 12 and the bottom layer 13 and it is joined thereto by glueing.

Furthermore, the semirigid element 8 of the present embodiment has a reduced extension with respect to the longitudinal 2 and transverse 3 dimensions of the elongated body 1.

In general terms, the extension of the semirigid element according to the invention is selected taking into account the flexibility of the material of which it is made. More specifically, if such material has a low deformability, as, e.g., Plexiglas, the semirigid element will not optimally fit the pelvis shape when the sanitary pad is worn. Hence, the extension of such element with respect to the entire elongated body should be limited, as it is in the present embodiment. In this instance, preferably, the longitudinal extension of the semirigid element will range from about 1/8 to 1/5 of the longitudinal dimension 2 of the pad 1, and the transverse extension thereof will be equal to about the 50% of the transverse dimension 3.

On the other hand, the semirigid element could have a greater extension provided that a more flexible material is selected (given, of course, the material's capability of not deforming under the pressure exerted thereon by the seams).

According to a variant of the present embodiment, the semirigid element has different stiffness in different directions, i.e., a greater stiffness according to the transversal direction of the elongated body and according to the orthogonal direction to, the elongated body, by virtue, e.g., of a composite structure with inner ribbings oriented according to said directions, or of other anisotropic structures.

It will also be appreciated that the present invention provides a further relevant advantage, i.e., the fact that the pad 1 is globally strenghtened and locally stiffened.

Such stiffening takes place at least at the vaginal opening, as in the present embodiment. This is the narrower pelvic region, where the pad is usually apt to form wrinkles. These wrinkles are not only bothersome to the user, but, above all, reduce the effectiveness of the sanitary pad itself by hiding to the organic discharge a portion of the absorbent surface thereof. Therefore, the stiffening due to the semirigid element 8 increases, by preventing a wrinkling thereof, the effectiveness of the sanitary pad.

Moreover, the arrangement of the semirigid element 8 at the vaginal opening allows it to effectively cooperate with the other layers in order to shield the garments from organic fluids. In fact, the semirigid element 8 represents a further impermeable layer, that improves the effectiveness of the pad 1 right where it is most needed, i.e., at the origin of the secretions.

Furthermore, the arrangement of the semirigid element 8 below the absorbent core 12 makes the former absolutely imperceptible to the user, whilst always carrying out said shielding function.

In the present embodiment, the pad 1 is of the type commonly known as 〈〈pantiliner〉〉. Of course, alternative embodiments can relate to sanitary pads of different type and shape. For instance, the inventive concept of the present invention could also effectively be applied to sanitary pads provided with side flaps.

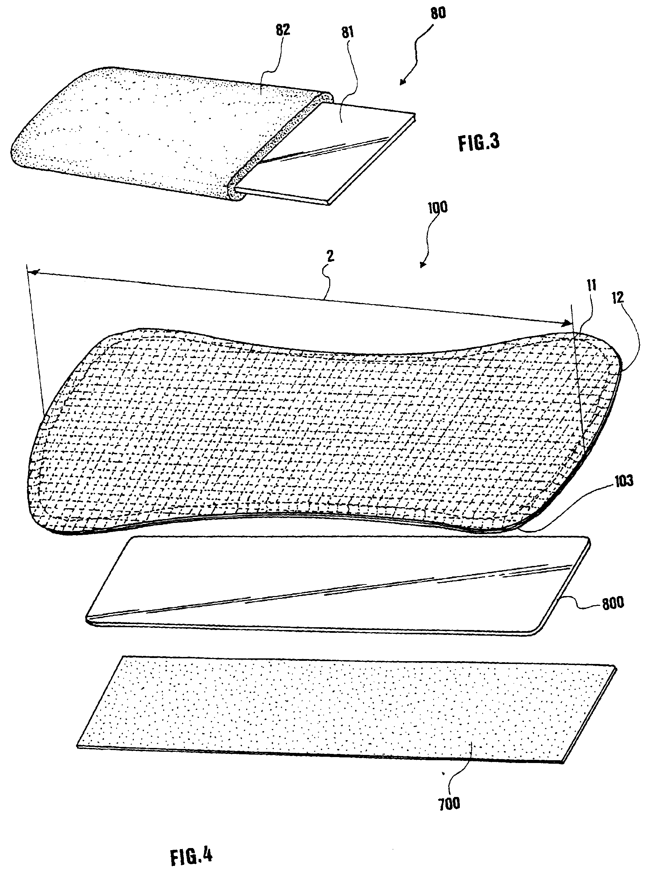

FIGS. 3 and 4 relate, respectively, to a second and to a third embodiment of the sanitary pad according to the invention. These further embodiments will be disclosed only in those aspects differentiating them from the first embodiment, and identical components will be designated with the same numberal reference already used.

FIG. 3 shows a second embodiment of the semirigid element according to the invention, indicated here with 80. According to this second embodiment, the semirigid element 80 has a stiff inner element 81, entirely enclosed within a soft envelope 82. The inner rigid element 81 can be made of, e.g., hard plastics, and the soft envelope 82 of sponge rubber.

In this second embodiment, the structure of the semiflexible element further enhances the imperceptibility of the element itself to the user.

Moreover, according to this second embodiment, even a highly stiff material could be used for the inner element 81. In fact, in case of an accidental breaking of the latter, the fragments thereof would anyhow remain safely confined within the envelope 82, with no risk of wounding the user.

Referring now to FIG. 4, according to a third embodiment, a sanitary pad, globally indicated with 100, has a top layer 11 and an absorbent core 12, adhered therebetween and wholly analogous to those already disclosed with reference to the first embodiment.

The sanitary pad 100 is further provided with a bottom layer 103 that is analogous to the one disclosed with reference to the first embodiment, except for the fact that the bottom layer 103 of this third embodiment does not have at the lower face thereof an adhesive apt to adhere to the panty.

Instead, to said lower face an elongated semirigid element 800, developing substantially over the entire longitudinal extension 2 of the pad 100 is fixed. Such semirigid element 800 can be fixed onto the bottom layer 103 by glueing.

The semirigid element 800 has, at the lower face thereof (not shown in the Figures), an adhesive apt to adhere the pad 100 to the panty.

The semirigid element 800 can be a thin light metal foil, e.g. of aluminium, and shape up the entire pad 100, stiffening it so as to perfectly fit it to the pelvic contour. In this way, once it is adhered to the panty, the pad also improves the wearability of the panty itself, preventing blemishes associated with the wrinkles which the panty could form in absence of the pad 100.

The pad 100 is provided to the user with a protective release band 700 analogous to that of the first embodiment, but in this case shaped so as to follow the contour of the semirigid element 800 of this third embodiment.

As above-mentioned, the semirigid element 800 provided in this third embodiment has a greater extension with respect to the previous two embodiments. According to a variant, the semirigid element could extend over the entire elongated body of the sanitary pad, replacing the bottom layer. Of course, in this case the semirigid element should be fluid-impermeable.

The present invention has hereto been described with reference to preferred embodiments thereof. It is understood that other embodiments afferent to the same inventive concept might be effected, all however comprised within the protective scope of the annexed claims.

## Claims

1. A sanitary pad (1; 100),
comprising a substantially flat elongated body (1; 100), having a longitudinal dimension (2) and a transverse dimension (3) larger than the thickness,
which elongated body has a multi-layer structure for absorbing organic fluids, said structure providing a fluid permeable top layer (11), apt to contact the user's skin, an intermediate absorbent core (12) and a fluid impermeable bottom layer (13, 103), apt to be adhered to a panty,
characterised in that said elongated body comprises
an element of a semirigid material (8; 80; 800), arranged in a substantially centred position with respect to said longitudinal and transverse dimensions.

2. The sanitary pad (1; 100) according to claim 1, wherein said element of a semirigid material (8; 80; 800) is made of plastics.

3. The sanitary pad (1; 100) according to claim 2, wherein said plastic material is selected from a group comprising Plexiglas and polyethylene.

4. The sanitary pad (1; 100) according to claim 1, wherein said element of a semirigid material (8; 80; 800) is made of a metallic foil.

5. The sanitary pad (1; 100) according to claim 4, wherein said foil comprises aluminium.

6. The sanitary pad (1; 100) according to claim 1, wherein said element of a semirigid material(8; 80) is made of a fluid-impermeable material.

7. The sanitary pad (1) according to claim 1, wherein said element of a semirigid material (8; 80) is arranged between said absorbent core (12) and said bottom layer (13).

8. The sanitary pad (100) according to claim 1, wherein said element of a semirigid material (800) is arranged below said bottom layer (103).

9. The sanitary pad (100) according to claim 8, wherein said element of a semirigid material (800) has, at a bottom face thereof, an adhesive (6) to adhere said sanitary pad to the panty.

10. The sanitary pad (1) according to claim 1, wherein said semirigid element (8; 80) has a reduced extension with respect to said longitudinal (2) and transversal (3) dimensions of said elongated body (1).

11. The sanitary pad (100) according to claim 1, wherein said element of a semirigid material (800) develops substantially over the entire longitudinal extension of said elongated body (100).

12. The sanitary pad (100) according to claim 1, wherein said element of a semirigid material (800) extends substantially along the entire elongated body (100) of said sanitary pad, forming said bottom layer (103).

13. The sanitary pad (100) according to claim 1, wherein said semirigid element (80) comprises a stiff inner element (81) and a soft envelope (82), said envelope completely enclosing said stiff element.
